(19) Europäisches Patentamt
European Patent Office
Office européen des brevets

(11) Publication number: **0 307 115 A1**

(12) # EUROPEAN PATENT APPLICATION

(21) Application number: 88307912.1

(51) Int. Cl.[4]: C07D 295/20 , A61K 31/535

(22) Date of filing: 26.08.88

(30) Priority: 07.09.87 GB 8721005
22.12.87 GB 8729796

(43) Date of publication of application:
15.03.89 Bulletin 89/11

(84) Designated Contracting States:
AT BE CH DE ES FR GB GR IT LI LU NL SE

(71) Applicant: IMPERIAL CHEMICAL INDUSTRIES PLC
Imperial Chemical House Millbank
London SW1P 3JF(GB)

(72) Inventor: Jones, Geraint
16 Packsaddle Park
Prestbury Cheshire SK10 4PU(GB)
Inventor: Taylor, David Christopher
3 Longdown Road
Congleton Cheshire CW12 4HQ(GB)

(74) Representative: Atkinson, John David et al
Imperial Chemical Industries PLC Legal Department: Patents PO Box 6 Bessemer Road
Welwyn Garden City Hertfordshire, AL7 1HD(GB)

(54) **Phenol esters.**

(57) A phenol ester of xamoterol of the formula:

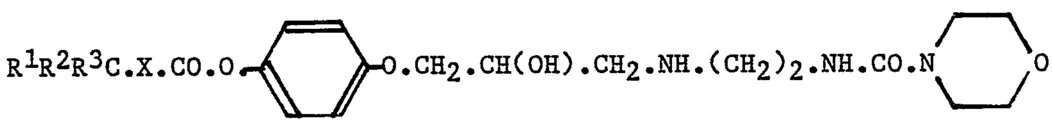

wherein X is a methylene radical or a direct bond,
$R^1$ is a hydrogen atom or an alkyl or alkenyl radical of 3 to 8 carbon atoms, and
$R^2$ and $R^3$, which may be the same or different, are each a 1-4C alkyl radical;
provided that

(i) when X is a direct bond, either $R^1$ is a hydrogen atom and $R^2$ and $R^3$ are each a 2-4C alkyl radical, or $R^1$ is a 3-8C alkyl or alkenyl radical and $R^2$ and $R^3$ are each a methyl radical; or

(ii) when X is a methylene radical, $R^1$ is an allyl radical or a 5-7C alkyl radical, and $R^2$ and $R^3$ are each a methyl radical; and the pharmaceutically acceptable acid addition salts thereof.

EP 0 307 115 A1

Xerox Copy Centre

# PHENOL ESTERS

This invention relates to phenol esters, and in particular it relates to esters of the phenol cardiac drug xamoterol, N-(2-[(RS)-2-hydroxy-3-(4-hydroxyphenoxy)-propylamino]ethyl)morpholine-4-carboxamide, which is a partial agonist for the $\beta$1 adrenergic receptors, and is used clinically in the management of congestive heart failure, and also has utility in the treatment of angina, and of obesity and related conditions.

When dosed orally in man, xamoterol is absorbed from the gastrointestinal tract only to the extent of about 9-10% of the administered dose, and of the drug which is absorbed, only about 50% is bioavailable, the remainder being effectively lost by conjugation. It is an object of this invention to provide phenol esters of xamoterol which are better absorbed gastro-intestinally than xamoterol itself; which, once absorbed, are hydrolysed to xamoterol, and thereby result in improved bioavailability of xamoterol, in man or in model species such as rat or dog; which do not themselves exert a significant effect at the $\beta$1 adrenergic receptors; and which possess no other undesirable pharmacological properties. Such esters of xamoterol may be expected to reduce inter-patient variability of response, or to reduce the required dose of drug, or to reduce the necessary frequency of dosing.

It is to be expected, of course, that the gastro-intestinal absorption characteristics of a drug can be modified by making chemical derivatives of the drug molecule, and in particular this would be expected for esters of drugs which possess a hydroxy substituent, since esters in general are more lipophilic than the parent hydroxy compound from which they are derived, and hence would naturally be expected to possess different partition, or absorption, characteristics. It is, however, believed to be completely unpredictable whether, regardless of such an ester's absorption properties, the pharmacological effect of administering such an ester will be similar to the effect obtained with the parent hydroxy drug molecule or not. If the ester is rapidly hydrolysed metabolically within the body, the pharmacological effect in a patient might be expected to be not very different from that obtained with the parent hydroxy compound, provided that the esterifying acid is innocuous at the dose level used and the blood level attained. If, however, the ester is not quickly hydrolysed metabolically, it might be expected that the pharmacological effect could be rather different from that obtained with the parent compound. More particularly, if an ester which is not quickly hydrolysed in the body, possesses significant $\beta$1 adrenergic receptor antagonist properties, the effect of administering such an ester to a patient with congestive heart failure could be fatal. However, the properties of esters of xamoterol at the $\beta$1 adrenergic receptors are unpredictable, and cannot be expected to bear any simple relationship to their gastrointestinal absorption properties.

European Patent Publication Number 172446A2 generically discloses, inter alia, some esters of xamoterol in which the phenolic hydroxy group of xamoterol is acylated with a lower acyl radical of 1 to 6 carbon atoms. This publication is addressed to compound for the treatment of blood flow disturbances, which act by bringing about an increase in the deformability of the erythrocytes. No such lower acyl derivatives of xamoterol are specifically disclosed, and there in no suggestion of the properties of such compounds at the $\beta$1 adenergic receptor, or of improved gastro-intestinal absorption for such compounds.

In fact, we have found that, among esters of xamoterol, there are many which are better absorbed gastro-intestinally than xamoterol, but which also possess significant undesirable $\beta$1 adrenergic receptor antagonist activity. There are also many others which possess little or no $\beta$1 adrenergic receptor antagonist activity, but are not significantly better absorbed gastro-intestinally than xamoterol itself. The present invention, therefore, provides esters of xamoterol which surprisingly are not only better absorbed gastro-intestinally than xamoterol itself, but also possess only about 10% or less of the $\beta$1 adrenergic receptor antagonist activity of xamoterol.

Thus, according to the invention, there is provided a phenol ester of xamoterol of the formula:-

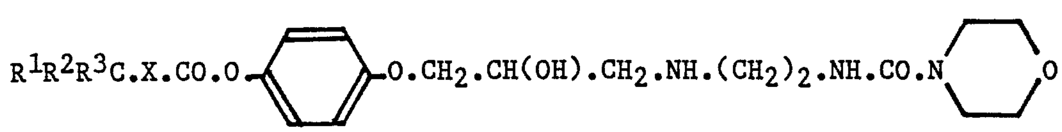

I

wherein X is a methylene radical or a direct bond,
$R^1$ is a hydrogen atom or an alkyl or alkenyl radical of 3 to 8 carbon atoms, and
$R^2$ and $R^3$, which may be the same or different, are each a 1-4C alkyl radical;
provided that

(i) when X is a direct bond, either $R^1$ is a hydrogen atom and $R^2$ and $R^3$ are each a 2-4C alkyl radical, or $R^1$ is a 3-8C alkyl or alkenyl radical and $R^2$ and $R^3$ are each a methyl radical; or

(ii) when X is a methylene radical, $R^1$ is an allyl radical or a 5-7C alkyl radical, and $R^2$ and $R^3$ are each a methyl radical; and the pharmaceutically acceptable acid addition salts thereof.

A suitable value for $R^1$ when it is a 3-8C alkyl radical is, for example, a propyl, isopropyl, butyl, isobutyl, sec-butyl, tert-butyl, pentyl, isopentyl, hexyl, isohexyl, heptyl, isoheptyl, octyl or iso-octyl radical.

A suitable value for $R^1$ when it is a 3-8C alkenyl radical is, for example, an allyl, butenyl, pentenyl, hexenyl or heptenyl radical, for example a 2- or 5-hexenyl or 2- or 6-heptenyl radical.

A suitable value for $R^2$ and $R^3$, when they are 1-4C alkyl radicals, is, for example, a methyl, ethyl, propyl, isopropyl, butyl, isobutyl, sec-butyl or tert-butyl radical.

A suitable pharmaceutically acceptable acid addition salt is, for example, the hydrochloride, hydrobromide, citrate, succinate, phosphate, acetate, sulphate, fumarate, trifluoroacetate, maleate, oxalate, toluene-p-sulphonte or methanesulphonate salt.

It is to be understood that this invention relates to the defined esters of both the racemate and the resolved (S)-isomer of xamoterol, which is the isomer having β1 adrenergic receptor partial agonist properties.

A particular preferred group of compounds of the invention comprises those compounds which $R^1$ is a 3-8C alkyl or 7-8C alkenyl radical, $R^2$ and $R^3$ are each a methyl radical, and X is a direct bond.

A further preferred group of compounds of the invention comprises those compounds wherein $R^1$ is a 5-7C alkyl or an allyl radical, $R^2$ and $R^3$ are each a methyl radical, and X is a methylene radical.

A further preferred group of compounds of the invention comprises those compounds wherein $R^1$ is a hydrogen atom, $R^2$ and $R^3$ are each a 3-4C alkyl radical, and X is a direct bond.

Particular preferred compounds of the invention are the 2,2-dimethylhexanoyl, 2,2,5-trimethylhexanoyl, 2,2-dimethyloctanoyl and 2,2,6-trimethylheptanoyl esters of xamoterol.

The xamoterol esters of the invention may be manufactured by any method known per se for the manufacture of analogous compounds. Thus, according to a further feature of the invention, there is provided a process for the manufacture of a xamoterol ester as defined above which comprises the reaction of xamoterol with a carboxylic acid of the formula $R^1R^2R^3C.X.COOH$, or with a reactive derivative thereof.

A suitable reactive derivative of such a carboxylic acid is, for example, an acid chloride, acid anhydride or mixed anhydride thereof. The reaction thereof with xamoterol is carried out under well-known conventional conditions in an appropriate solvent.

Certain of the carboxylic acids and/or reactive derivatives thereof used as starting materials in the above process are novel compounds, which may be obtained as follows.

Those carboxylic acids wherein $R^2$ and $R^3$ are methyl radicals and X is a direct bond may be manufactured by reacting the appropriate alkyl bromide with isobutyric acid in the presence of a strong base, such as n-butyl-lithium, in conventional manner. The carboxylic acids thus obtained may be converted to a corresponding reactive derivative thereof, for example the acid chloride, by reaction thereof with thionyl chloride.

Those carboxylic acids wherein $R^2$ and $R^3$ are methyl radicals and X is a methylene radical may be manufactured by reacting diethyl isopropylidenemalonate with a Grignard reagent prepared from the appropriate alkyl bromide, followed by hydrolysis and decarboxylation, for example with potassium hydroxide or sodium hydroxide. The carboxylic acids thus obtained may be converted to a corresponding reactive derivative thereof, for example the acid chloride, by reaction theoreof with thionyl chloride.

The gastro-intestinal absorption of a compound in the rat was determined as follows:-

Each experiment was carried out to study two test compounds, each in six rats, with an internal control of xamoterol in six additional rats. The animals were fasted overnight prior to administration of the test compounds, and for 4 hours after. During this time, and for the subsequent 24 hours, they were allowed free access to drinking water containing 10% of dextrose to increase the volume of urine excreted. A solution of a test compound or of xamoterol fumarate, in either case equivalent to 1mg of xamoterol base, was adminstered orally to male rats weighing approximately 200 g, as a solution in 0.5ml of distilled water. After being dosed, the animals were placed individually in "Metabowls", cages which collect all urine excreted. Four hours after the compounds had been dosed, when food was resupplied, each animal received a loading dose of 5ml of 10% dextrose, administered by gavage. All urine voided during 0-12 and 12-24 hours after compound dosing was collected, each sample was diluted with distilled water to a standard volume, and an aliquot stored at -20° for radio-immune assay. Since xamoterol is eliminated in urine largely as the glucuronide, the urine samples, (further diluted when necessary), were treated with 60 units of β-glucuronidase Type IX (Sigma, No. G-3510) per ml of sample, and incubated overnight at 37°,

then the xamoterol content was determined by the radio-immunoassay method for this compound. Standard solutions of the glucuronide conjugate of xamoterol in dilute, blank rat urine at concentrations equivalent to 25 and 50ng of xamoterol base per ml were included as internal controls in the glucuronidase hydrolysis step.

The concentration of xamoterol in the urine from rats treated with a xamoterol derivative, as compared with the concentration in the urine from rats treated with xamoterol itself, gives a measure of the compounds' relative absorptions from the gastro-intestinal tract.

The gastro-intestinal absorption of compounds in the dog was determined as follows:-

The test compound was mixed with an equal weight of lactose and filled into gelatin capsules, so that each capsule contained a dose of test compound equivalent, on a weight basis, to 50mg of xamoterol base. The determination was carried out as a crossover experiment in 6 dogs, with xamoterol itself included as a control in each experiment. Blood samples were withdrawn at intervals after the animals had been dosed with the capsules of test compound, and the plasma separated and immediately diluted with pH6.8 phosphate buffer. An aliquot of each sample was heated at 100° for 5 minutes, and the concentration of xamoterol measured by radio-immune assay. The area under the plasma concentration versus time curve (0-24h) was used to provide a measure of the amount of compound absorbed. Comparison of this value with the value obtained by dosing xamoterol gave a measure of the absorption of test compound relative to xamoterol.

The β1 adrenergic receptor antagonist activity of a compound is indicated by its estimated $pA_2$ value, which is determined as follows:-

Spontaneously beating rat right atria were attached to bipolar recording electrodes and suspended in a bath containing Krebs solution. The Krebs solution was maintained at 32° and bubbled with 95% oxygen and 5% carbon dioxide. After 90 minutes equilibration time, a cumulative dose-response curve to isoprenaline was constructed, the responses being expressed as a percentage of the maximum increase in atrial rate. The tissues were then washed every 15 minutes for a period of 1 hour until they returned to their original rates of beating. The compound to be tested was then added to the bath at the required concentration for a contact time of one hour, after which a second cumulative dose-response curve to isoprenaline was constructed. A $pA_2$ value was then estimated for the test compound from the (dose) ratio of the isoprenaline $EC_{50}$ values as follows:

est. $pA_2$ = 1og (dose ratio -1)-(1og molar concentration of test compound).

The compounds of the invention are absorbed in either the rat or the dog, as determined by the procedures described above, to an extent which is at least 50% greater than that obtained with xamoterol itself, and they have estimated $pA_2$ values of less than 7.3. Preferred compounds have an estimated $pA_2$ value as low as possible, combined with gastro-intestinal absorption which is as high as possible.

Thus, according to the invention, there is provided a pharmaceutical composition comprising a xamoterol ester as defined above together with a pharmaceutically acceptable diluent or carrier therefor.

The composition of the invention may be manufactured in conventional manner, using conventional pharmaceutical excipients.

Preferred compositions according to the invention are solid formulations for oral use, for example tablets or capsules, and preferred such tablets or capsules contain from 40mg to 400mg of a xamoterol ester of the invention.

The invention is illustrated, but not limited by the following Examples. Temperatures are given in °C, and melting points are uncorrected.

## Example 1

N-(2-[(RS)-2-Hydroxy-3-(4-hydroxyphenoxy)propylamino]ethyl)morpholine-4-carboxamide fumarate (xamoterol, 6.0g, 0.015 moles) was suspended in methylene dichloride (35ml) and trifluoroacetic acid (15ml) was added with stirring. A solution of 2,2-dimethylhexanoyl chloride (2.94, 0.018 moles) in methylene dichloride (10ml) was added dropwise at room temperature. After the addition was complete, the reaction mixture was stirred for a further 2 h. The methylene dichloride and excess trifluoroacetic acid were evaporated under reduced pressure, and the residue was partitioned between ethyl acetate and saturated sodium bicarbonate solution (4 x 20ml). The aqueous solution was separated, saturated with further solid sodium bicarbonate and extracted twice with ethyl acetate. The combined organic extracts were washed with water and then brine, dried over magnesium sulphate and evaporated to dryness to yield an oil (9.7g). The crude product was freed from any unreacted phenolic starting material by chromatography on alumina (Fluka 507C, 250g, 100-200 mesh) and the pure product was eluted with methylene dichloride/methanol

(19:1, v/v). The free base was converted to the trifluoroacetate salt, using the stoichiometric amount of the acid in methanol solution. The solvent was removed and the residue was crystallised from isopropyl acetate, to give N-(2-[(RS)-3-(4-[2,2-dimethylhexanoyloxy]phenoxy)-2-hydroxypropylamino]ethyl)morpholine-4-carboxamide trifluoroacetate, mp 100-101 °C.

**Examples 2-18**

The process described in Example 1 was repeated, using the appropriate acid chloride in place of 2,2-dimethylhexanoyl chloride, to give the following compounds:-

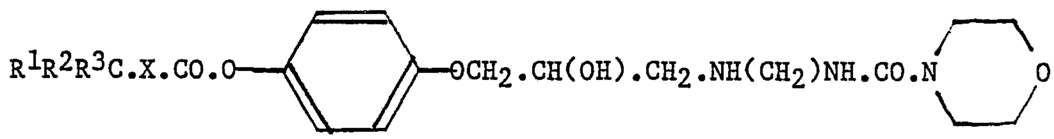

| Ex | $R^1$ | $R^2$ | $R^3$ | X | M.p. | Footnotes |
|---|---|---|---|---|---|---|
| 2 | n-propyl | methyl | methyl | – | 117–118 | 1 |
| 3 | isopentyl | " | " | – | 122–124 | 2 |
| 4 | n-hexyl | " | " | – | 132–134 | 1 |
| 5 | isohexyl | " | " | – | 138–140 | 2 |
| 6 | n-heptyl | " | " | – | 119–121 | 1 |
| 7 | n-octyl | " | " | – | 109–111 | 2 |
| 8 | 2-heptenyl | " | " | – | 125–128 | 1 |
| 9 | 5-hexenyl | " | " | – | 128–130 | 1 |

continued.....

......continued

| Ex | $R^1$ | $R^2$ | $R^3$ | X | M.p. | Footnotes |
|---|---|---|---|---|---|---|
| 10 | 2-hexenyl | " | " | - | 134-136 | 1 |
| 11 | allyl | " | " | $CH_2$ | 98-100 | 3 |
| 12 | n-hexyl | " | " | " | 149-152 | 4,5 |
| 13 | n-heptyl | " | " | " | 119-121 | 6 |
| 14 | isopentyl | " | " | " | 129-132 | 6 |
| 15 | isohexyl | " | " | " | 123-125 | 6 |
| 16. | n-propyl | n-propyl | H | - | 123-125 | 1 |
| 17 | n-butyl | n-butyl | " | - | 110-112 | 1 |
| 18 | isopropyl | isopropyl | " | - | 102-104 | 6 |

Footnotes

1. Crystallised from ethyl acetate.
2. Crystallised from isopropyl acetate.
3. Crystallised from tert-butyl acetate.
4. Oxalate salt.
5. Crystallised from ethanol.
6. Crystallised from isopropyl alcohol.

The acid chlorides which are used in the above Examples were manufactured from the corresponding carboxylic acids as follows:-

The acid (1 mole) was mixed at room temperature with thionyl chloride (1.05 moles) and a catalytic amount of dimethylformamide, and the mixture was stirred for 2 days. In the case of sterically hindered acids, an excess of thionyl chloride was used, and the reaction mixture was heated under reflux for 17h. The excess of thionyl chloride was removed by azeotroping three times with toluene. In the case of olefinic acids, oxalyl chloride was used in preference to thionyl chloride. The crude acid chlorides were used directly, without distillation, in the acylation procedure described above for the preparation of xamoterol esters.

Certain of the carboxylic acids, used in the preparation of the above acid chlorides, are novel compounds and were obtained as follows:-

(i) For those compounds wherein $R^2$ and $R^3$ are methyl and X is a direct bond:

A mixture of N,N-di-isopropylamine (45.5g, 63ml, 0.45 mole) and freshly distilled tetrahydrofuran (300ml) was cooled to -50° and n-butyl-lithium (300ml, 0.485 mole) was added. The mixture was stirred at -20° for 1h, then isobutyric acid (19.8g, 20.8ml, 0.225 mole) was added and the mixture was stirred and allowed to warm to room temperature over 4h. The appropriate alkyl bromide (0.15 mole) was added at room temperature, and the mixture was stirred overnight, then acidified with concentrated hydrochloric acid (30ml) in water (150ml). The upper organic layer was separated, and the aqueous layer was extracted 5 times with diethyl ether (75ml). The combined organic extracts were washed with brine (4 x 75ml) and dried, the solvents were evaporated under reduced pressure, and the residual oil was purified by vacuum

distillation to give the following acids:-
2,2,5-Trimethylhexanoic acid (for Example 4), bp 83-85° (0.3 mm Hg.) NMR in $CDCl_3$: δ 0.86(6H,d); 1.18-(8H, complex); 1.50(3H, m);
2,2,6-Trimethylheptanoic acid (for Example 6), bp 103-106° (0.2 mm Hg). NMR in $CDCl_3$: δ 0.90(6H,d); 1.20(s); 1.1-1.7(13H, complex);
(E)-2,2-dimethyl-4-nonenoic acid (for Example 9), bp. 112-114° (0.1 mm Hg.) NMR in $CDCl_3$: δ 0.9(3H,t); 1.16(6H,s); 1.16-1.45(4H,m); 1.98(2H,m); 2.21(2H,m); 5.38(2H,m);
(E)-2,2-dimethyl-7-octenoic acid (for Example 10), bp. 100-102° (0.1 mm Hg.) NMR in $CDCl_3$: δ 1.20(6H,s); 1.2-1.7(6H, complex); 2.08(2H,m); 4.8-5.1(2H, complex); 5.8(1H,m);
(E)-2,2-dimethyl-4-octenoic acid (for Example 11), bp 112-114° (0.1mm Hg.) NMR in $CDCl_3$: δ 0.84(3H,t); 1.16(6H,s); 1.16-1.50(2H,m); 1.96(2H,m); 2.20(2H,d); 5.38(2H,m).

ii) For those compounds wherein $R^2$ and $R^3$ are methyl and X is methylene:

4-Methylpentylmagnesium bromide was prepared from 4- methylpentyl bromide (59.7g, 0.33 mole) and magnesium (8.02g 0.33 g atoms) in diethyl ether (150ml). The Grignard solution so obtained was then diluted with diethyl ether (400ml), and to this solution was added a solution of diethyl isopropylidenemalonate (60g, 0.30 mole) in diethyl ether (50ml) at such a rate as to maintain a steady reflux. After the addition was complete, the reaction mixture was heated under reflux for a further 1.5h. The reaction complex was then decomposed by the controlled addition of dilute sulphuric acid (10% w/v. 500 ml). The organic layer was separated, and the aqueous layer was extracted twice with diethyl ether. The combined organic layers were washed with water and then brine, dried, and evaporated to dryness to yield a mobile oil. Distillation gave a fraction bp 108-140° C/0.2mm Hg. The distilled oil was dissolved in ethanol (200ml), and the solution was heated under reflux with a solution of potassium hydroxide (20.6g) in water (50ml) for 20h. The reaction mixture was cooled, diluted with water (250ml) and extracted twice with diethyl ether. The basic, aqueous solution was acidified with concentrated hydrochloric acid (30ml), and extracted 3 times with diethyl ether. The combined ether extracts were washed with brine, dried, and evaporated to dryness, and the residue was distilled through a short Vigreux column to give 3,3,7-trimethyloctanoic acid, bp. 146-8° (0.1 mm Hg.) NMR in $CDCl_3$: δ 0.93(6H,d); 1.10(6H,s); 1.2-1.8(9H, complex); 2.30(2H,s).

In a similar manner, there was obtained 3,3,6-trimethylheptanoic acid, bp. 128-130° (0..2 mm Hg,) NMR in $CDCl_3$: δ 0.92(6H, d); 1.05(6H,s); 1.2-1.6(5H, complex); 2.24(2H,s).

## Example 19

The process described in Example 1 was repeated, using N-(2-[(S)-2-hydroxy-3-(4-hydroxyphenoxy)-propylamino]ethyl)morpholine-4-carboxamide in place of the (R,S) racemate, and 2-propylpentanoyl chloride in place of 2,2-dimethylhexanoyl chloride to give N-(2-[(S)-2-hydroxy-3-(4-[2-propylpentanoyloxy]phenoxy)-propylamino]ethyl)morpholine-4-carboxamide trifluoroacetate, crystallised from ethyl acetate, m.p. 107-110°, $[\alpha]^{D}_{25}$ = -8.8° (C = 1.15g/100ml in methanol. The (S)-isomer of xamoterol, used as the starting material in the above process, was obtained as follows:-

Sodium hydride (0.44g) was washed with dry hexane (3 x 3ml) and suspended in dimethyl formamide (5ml) under a nitrogen atomosphere. The suspension was cooled at 0-5°, and a solution of p)-benzyloxyphenol (1.75, 0.00875 moles) in dimethyl-formamide (5ml) was added dropwise. The mixture was stirred at 0-5° for 10 minutes and then a solution of (2S)-2,3-epoxypropyl p-toluenesulphonate (2.0g, 0.00877 moles) in dimethylformamide (2.5 ml) was added dropwise. The reaction mixture was allowed to warm to room temperature and stirred for a further 2h. The reaction mixture was then diluted with water (20ml) and extracted with ethyl acetate (4 x 50ml). The ethyl acetate extracts were combined, washed with water and then brine, dried, filtered and evaporated to dryness to give a white crystalline solid (3.55g). The crude product was filtered through silica gel (Merck ART 9385, 150g) using toluene ethyl acetate (19:1 v/v) as the eluent. Collection of the appropriate fractions and evaporation gave a solid which was crystallised from cyclohexane to give (2S)-1-(p-benzyloxyphenoxy)-2,3-epoxypropane, mp. 75-6° C. $[\alpha]^{20}_{D}$ = +8.6 (c = 1.02 in methanol).

A solution of (2S)-1-(p-benzyloxyphenoxy)-2,3-epoxypropane (2.0g, 0.0078 mole) in isopropyl alcohol (20ml) was treated successively with a solution of sodium hydroxide (0.8g) in water (5ml) and a solution of 4-(N-2-aminoethylcarbamoyl)-morpholine hydrogen sulphate (4.45g, 0.01 mole) in isopropyl alcohol (12ml) and water (5ml). The reaction mixture was heated on a steam-bath for 2h, cooled and neutralisd to pH8 with

dilute sulphuric acid. The solution was extracted with ethyl acetate, and the ethyl acetate extract was washed with water, dried, concentrated to approximately 25ml. and allowed to crystallise. The solid which separated was collected by filtration, washed with ethyl acetate and dried. Crystallisation of an aliquot of the solid gave N-(2-[(2S)-3-(4-benzyloxyphenoxy)-2-hydroxypropylamino]ethyl)morpholine-4-carboxamide, mp. 108-10°, $[\alpha]^{20}_D$ = -3.8 (c = 1.19 in methanol.

A solution of N-(2-[(2S)-3-(4-benzyloxyphenoxy)-2-hydroxypropylamino]ethyl)morpholine-4-carboxamide (1.0g) in ethanol (5ml) and acetic acid (0.35ml) was treated with 5% palladium-on-carbon catalyst and hydrogenated at room temperature and pressure. After hydrogen uptake had stopped (165mins), the catalyst was filtered off and a solution of fumaric acid (0.15g) in ethanol (3ml) was added. On standing, crystals separated which were collected by filtration, washed with ethanol and dried, to give the required (S)-xamoterol as its fumarate salt, m.p. 175-6°, $[\alpha]^{20}_D$ = -8.7° (c = 1.99 in water).

## Claims

1. A phenol ester of xamoterol of the formula:

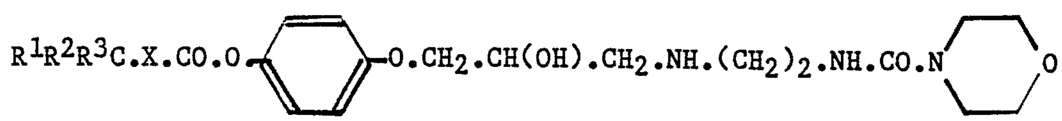

wherein X is a methylene radical or a direct bond,
$R^1$ is a hydrogen atom or an alkyl or alkenyl radical of 3 to 8 carbon atoms, and
$R^2$ and $R^3$, which may be the same or different, are each a 1-4C alkyl radical;
provided that

(i) when X is a direct bond, either $R^1$ is a hydrogen atom and $R^2$ and $R^3$ are each a 2-4C alkyl radical, or $R^1$ is a 3-8C alkyl or alkenyl radical and $R^2$ and $R^3$ are each a methyl radical; or

(ii) when X is a methylene radical, $R^1$ is an allyl radical or a 5-7C alkyl radical, and $R^2$ and $R^3$ are each a methyl radical; and the pharmaceutically acceptable acid addition salts thereof.

2. A phenol ester as claimed in claim 1 wherein $R^1$ is a propyl, isopropyl, butyl, isobutyl, sec-butyl, tert,butyl, pentyl, isopentyl, hexyl, isohexyl, heptyl, isoheptyl, octyl, iso-octyl, allyl, butenyl, pentenyl, hexenyl or heptenyl radical and $R^2$ and $R^3$, which may be the same or different, are each a methyl, ethyl, propyl, isopropyl, butyl, isobutyl, sec-butyl or tert-butyl radical.

3. A phenol ester as claimed in claim 1 wherein $R^1$ is a 3-8C alkyl or 7-8C alkenyl radical $R^2$ and $R^3$ are each a methyl radical and X is a direct bond.

4. A phenol ester as claimed in claim 1 wherein $R^1$ is a 5-7C alkyl or an allyl radical, $R^2$ and $R^3$ are each a methyl radical and X is a methylene radical.

5. A phenol ester as claimed in claim 1 wherein $R^1$ is a hydrogen atom, $R^2$ and $R^3$ are each a 3-4C alkyl radical and X is a direct bond.

6. A phenol ester as claimed in claim 1 which is the 2,2-dimethylhexanoyl, 2,2,5-trimethylhexanoyl, 2,2-dimethyloctanoyl or 2,2,6-trimethylheptanoyl ester of xamoterol.

7. A process for the manufacture of a phenol ester of the formula I which comprises the reaction of xamoterol with a carboxylic acid of the formula $R^1R^2R^3C.X.COOH$, or with a reactive derivative theoreof.

8. A pharmaceutical composition comprising a phenol ester of xamoterol, as claimed in claim 1, together with a pharmaceutically acceptable diluent or carrier therefor.

European Patent Office

# EUROPEAN SEARCH REPORT

Application Number

EP 88 30 7912

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | EP-A-0 085 286 (AKTIEBOLAGET HÄSSLE)<br>* Page 6, line 30 - page 8, claims *<br>--- | 1-5,7-8 | C 07 D 295/20<br>A 61 K 31/535 |
| Y | US-A-4 080 471 (CARLSSON et al.)<br>* Column 8, line 60 - column 9, line 2; claims *<br>--- | 1-5,7-8 | |
| A | WO-A-8 301 772 (AMERICAN HOSPITAL SUPPLY CORP.)<br>* Claims *<br>--- | 1-8 | |
| A,D | EP-A-0 172 446 (BOEHRINGER)<br>* Whole document *<br>--- | 1-8 | |
| A | EP-A-0 004 532 (MERCK)<br>* Claims *<br>----- | 1-8 | |
| | | | **TECHNICAL FIELDS SEARCHED (Int. Cl.4)** |
| | | | C 07 C 295/00<br>A 61 K 31/00 |

The present search report has been drawn up for all claims

| Place of search | Date of completion of the search | Examiner |
|---|---|---|
| THE HAGUE | 05-12-1988 | HELPS I.M. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)